Europäisches Patentamt

European Patent Office

Office européen des brevets

(19)

(11) Numéro de publication : **0 448 896 A1**

(12) # DEMANDE DE BREVET EUROPEEN

(21) Numéro de dépôt : **90403706.6**

(51) Int. Cl.⁵ : **G01N 29/18**

(22) Date de dépôt : **20.12.90**

(30) Priorité : **21.12.89 FR 8917009**

(43) Date de publication de la demande :
**02.10.91 Bulletin 91/40**

(84) Etats contractants désignés :
**AT BE CH DE DK ES GB GR IT LI LU NL SE**

(71) Demandeur : **CENTRE D'ETUDES ET DE RECHERCHES DE L'INDUSTRIE DU BETON MANUFACTURE, CERIB B. P. 59 F-28232 Epernon (FR)**

(72) Inventeur : **Sanchez, Alain P.
26 rue des Gardes
F-28230 Epernon (FR)**
Inventeur : **Beinish, Hervé R.
16, rue de la Garenne au Moine
F-28230 Epernon (FR)**
Inventeur : **Randriamazaoro, Jean D.
11, rue des Craviers de Ponceau
F-28130 Maintenon (FR)**

(74) Mandataire : **Levesque, Denys et al
Cabinet Beau de Loménie 55, rue d'Amsterdam
F-75008 Paris (FR)**

(54) **Procédé et dispositif de contrôle non destructif pour des éléments en béton.**

(57) Dispositif de contrôle non destructif pour des éléments en béton, comprenant un moule (1) ; des transducteurs à ultrasons (E, R) fixés sur le moule, un transducteur-émetteur (E) étant placé sur une paroi du moule faisant face à une autre paroi supportant des transducteurs-récepteurs (R1, R2, R3) ; un dispositif de fixation associé à chaque transducteur afin de le maintenir contre une des parois du moule (1) ; un ensemble électronique (4) relié à chacun des transducteurs et destiné à déterminer le temps de propagation des ondes se propageant dans le béton entre le transducteur-émetteur (E) et les transducteurs-récepteurs (R1, R2, R3) ; un calculateur programmable relié en entrée à l'ensemble électronique (4) et en sortie à un dispositif de visualisation (8), destiné à traiter lesdits temps de propagation des ondes afin de fournir en continu l'évolution des caractéristiques de l'élément en béton.

EP 0 448 896 A1

FIG.1

# PROCEDE ET DISPOSITIF DE CONTROLE NON DESTRUCTIF POUR DES ELEMENTS EN BETON

La présente invention concerne un procédé et un dispositif de contrôle non destructif pour des éléments en béton, plus précisément destinés à déterminer une caractéristique mécanique liée à la vitesse de propagation d'ondes ultrasonores se propageant dans le béton, à différents moments de leur fabrication.

Il existe de façon connue des dispositifs pour étudier les matériaux à l'aide d'ondes ultrasonores, c'est-à-dire pour le contrôle non destructif de matériaux.

Une application particulière est divulguée dans le document FR 2 396 294 qui concerne le contrôle in situ de matériaux de construction ou de roches pour déceler dans ces matériaux différents types de défauts, grâce à la mesure de la vitesse de propagation des oscillations ultrasonores dans lesdits matériaux. Ce document propose plus précisément une amélioration du contrôle prédéfini en permettant un fonctionnement correct et fiable du dispositif même en présence de bruits acoustiques.

Pour le contrôle des produits manufacturés en béton tels que visés par la présente invention, on effectue habituellement des essais destructifs sur des éprouvettes en un même matériau que celui à contrôler. Cependant, ce contrôle présente certains inconvénients: les éprouvettes ainsi réalisées coûtent cher, elles sont en nombre limité, et de plus elles ne sont pas nécessairement représentatives du matériau à contrôler. En effet, les conditions de fabrication, et en particulier les conditions de vibration des éprouvettes en béton, sont différentes de celles d'un élément de plus grande dimension qui comporte souvent des zones plus ou moins vibrées. L'éprouvette ne représentera donc pas nécessairement la zone la plus vibrée ou la zone la moins vibrée de l'élément, l'éprouvette ne pourra en aucun cas tenir compte des dispersions dans la constitution du matériau.

En outre, le fait qu'il n'existe, essentiellement pour des raisons de coûts, qu'un nombre limité d'éprouvettes implique donc un contrôle à certains moments seulement de la fabrication du matériau. Il faut donc procéder par interpolation pour représenter, en continu, l'évolution du matériau dans le temps; cette méthode peut s'avérer dangereuse, car il peut se produire des incidents au cours du cycle de production, entre deux contrôles. Ces incidents par conséquent ne seront pas nécessairement détectés avec ce type de contrôle destructif.

La présente invention permet d'assurer un suivi en continu de l'évolution de la vitesse du son dans le matériau et indirectement de sa résistance.

De plus, l'inertie thermique d'un petit élément tel qu'une éprouvette est bien différente de celle d'un élément en béton de grande dimension. Par conséquent, les conditions mêmes de fabrication d'une éprouvette sont différentes de celles de la fabrication d'un élément en grandeur réelle. Certaines caractéristiques mécaniques du matériau, et notamment le durcissement, seront donc différentes, ce qui signifie que l'éprouvette n'est pas toujours représentative du produit à contrôler.

Dans le cas particulier du contrôle de béton précontraint, il est essentiel de connaître la résistance atteinte par le béton au moment du relâchement des armatures, après le cycle d'étuvage. Celle-ci a une incidence sur l'adhérence entre l'acier et le béton et donc sur les caractéristiques finales de l'élément précontraint.

Il s'est donc avéré nécessaire de réaliser un dispositif de contrôle non destructif du type mentionné au début, qui comprend, conformément à la présente demande, outre un moule destiné à contenir le béton:

– des transducteurs à ultrasons fixés sur le moule, un transducteur-émetteur étant placé sur une paroi du moule faisant face à une autre paroi supportant plusieurs transducteurs-récepteurs;

– un dispositif de fixation associé à chaque transducteur afin de le maintenir contre une des parois du moule;

– un ensemble électronique relié à chacun des transducteurs et destiné à déterminer les temps de propagation des ondes se propageant dans le béton entre le transducteur-émetteur et les transducteurs-récepteurs;

– un calculateur programmable relié en entrée à l'ensemble électronique et en sortie à un dispositif de visualisation, destiné à traiter lesdits temps de propagation des ondes afin de fournir en continu des données relatives à l'évolution des caractéristiques de l'élément en béton, dont notamment sa résistance mécanique.

Selon une caractéristique de la présente invention, plusieurs transducteurs-récepteurs sont fixés en des endroits prédéterminés sur une première paroi du moule et un seul émetteur est prévu sur la paroi métallique faisant face à ladite première paroi.

Selon une autre caractéristique conforme à la présente invention, l'ensemble électronique mesure (et enregistre) successivement les temps stabilisés de parcours des trains d'ondes entre l'émetteur et chacun des récepteurs.

Conformément à une autre particularité de l'invention, le calculateur programmable détermine la vitesse de propagation de l'onde entre l'émetteur et chacun des récepteurs, élimine les valeurs de vitesse n'appartenant pas à une plage prédéfinie (valeurs aberrantes), calcule la vitesse moyenne des vitesses non éliminées, à des intervalles de temps prédéterminés, et ce jusqu'à l'obtention d'une valeur de vitesse moyenne prédéfinie.

Avantageusement les transducteurs sont fixés sur le moule de façon que leur surface "active" soit située dans le plan de la surface interne du moule métallique. Grâce à des dispositifs de fixation constitués chacun:

- d'un corps tubulaire entourant et positionnant chacun desdits transducteurs;
- d'une étoupe coaxiale au corps, entourant en partie le transducteur et axialement en appui sur ledit corps;
- d'un presse-étoupe tubulaire coaxial au corps, et interposé entre ladite étoupe et un bouchon de serrage vissé sur ledit corps.

De préférence, un film silicone est placé sur chaque transducteur pour être utilisé comme couplant.

La présente demande concerne également un procédé de contrôle non destructif de produits en béton utilisant le dispositif qui vient d'être décrit, et comportant les étapes suivantes:

a) - placer en des endroits prédéterminés sur un moule destiné à recevoir le béton, plusieurs transducteurs à ultrasons, l'un étant émetteur et les autres étant récepteurs;

b) - faire émettre par ledit transducteur-émetteur des trains d'ondes successifs;

c) - mesurer successivement les temps de propagation stabilisés entre l'émetteur et chaque récepteur;

d) - convertir les temps en vitesse;

e) - traiter lesdites valeurs de vitesse;

f) - établir une corrélation entre les valeurs de vitesse et certaines caractéristiques physiques du béton;

- répéter les étapes b) à f) jusqu'à obtention d'une caractéristique physiques prédéterminées du béton.

De façon caractéristique, le traitement des valeurs de vitesse consiste à:

- comparer chaque vitesse de propagation à une valeur de seuil;
- éliminer la ou les vitesses supérieures à un certain seuil, et
- s'il reste plusieurs valeurs de vitesse, en faire la moyenne arithmétique, sinon conserver ladite valeur;
- comparer ladite moyenne ou ladite valeur à une valeur prédéfinie.

D'autres caractéristiques et avantages de la présente invention apparaîtront mieux à la lecture de la description qui va suivre, faite en référence aux dessins annexés sur lesquels:

- la figure 1 est un schéma de principe du dispositif de contrôle non destructif selon la présente invention;
- la figure 2 est une coupe d'un dispositif de fixation destiné à maintenir un transducteur à ultrasons sur une paroi du moule;
- la figure 3 représente un schéma de principe de l'ensemble de traitement conforme à la présente invention;

- la figure 4 est un schéma montrant le principe de la méthode de détermination de la vitesse du son dans des éléments en béton.

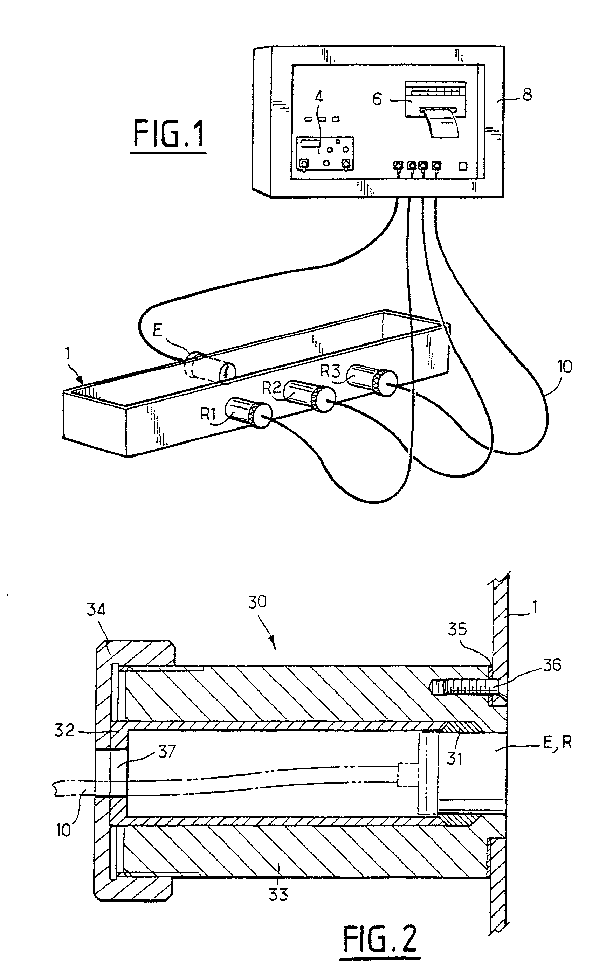

La figure 1 montre les composants essentiels du dispositif de la présente invention.

Ce dispositif est constitué d'un moule 1 dans lequel du béton frais est coulé et vibré. Les machines nécessaires au coulage et à la vibration du béton ne sont bien entendu pas représentées sur la figure.

Le moule 1 est équipé de transducteurs à ultrasons: un émetteur E est fixé sur une des parois verticales du moule, tandis que plusieurs (trois - R1, R2, R3 - dans le mode de réalisation décrit ici) récepteurs R sont placés sur la paroi opposée du moule. Ces transducteurs sont fixés grâce à des dispositifs de fixation qui seront décrits plus loin en détail.

Chaque transducteur E, R à ultrasons est donc positionné sur les parois du moule 1 grâce à des dispositifs spécifiques 30 ayant notamment pour fonctions:

- de protéger les transducteurs et les câbles 10 de liaison des effets de la température;
- d'éviter la propagation des ondes ultrasonores dans les parois du moule 1;
- de positionner et de maintenir les transducteurs dans le plan des parois internes du moule afin d'assurer un contact satisfaisant entre les transducteurs et le béton.

Pour réaliser ces fonctions, les dispositifs 30 sont constitués, comme il est visible sur la figure 2:

- d'un corps cylindrique 33;
- d'une étoupe 31 de préférence en Téflon qui entoure le transducteur E, R et est axialement comprimée entre le corps 33 et un presse-étoupe 32;
- d'un bouchon de serrage 34, par exemple en aluminium, dont le taraudage est vissé sur un filetage à l'extrémité du corps 33 non en contact avec le moule 1.

Afin de pouvoir adapter les dispositifs de fixation 30 sur des moules 1 d'épaisseurs différentes, des petites cales annulaires telles que 35 peuvent être interposées entre le corps 33 des dispositifs 30 et la paroi externe du moule 1.

Des vis 36 assurent le maintien des corps 33 des dispositifs 30 contre la paroi externe du moule 1.

Un orifice central 37 ménagé dans le presse-étoupe 32 et dans le bouchon de serrage 34 permet le passage des câbles de liaison 10.

Ces dispositifs de fixation 30 permettent un réglage très précis de la position du transducteur.

En outre, un film silicone non représenté peut être interposé entre la face active du transducteur et l'intérieur du moule. Ce film améliore encore le couplage entre les transducteurs et le matériau à l'intérieur du moule.

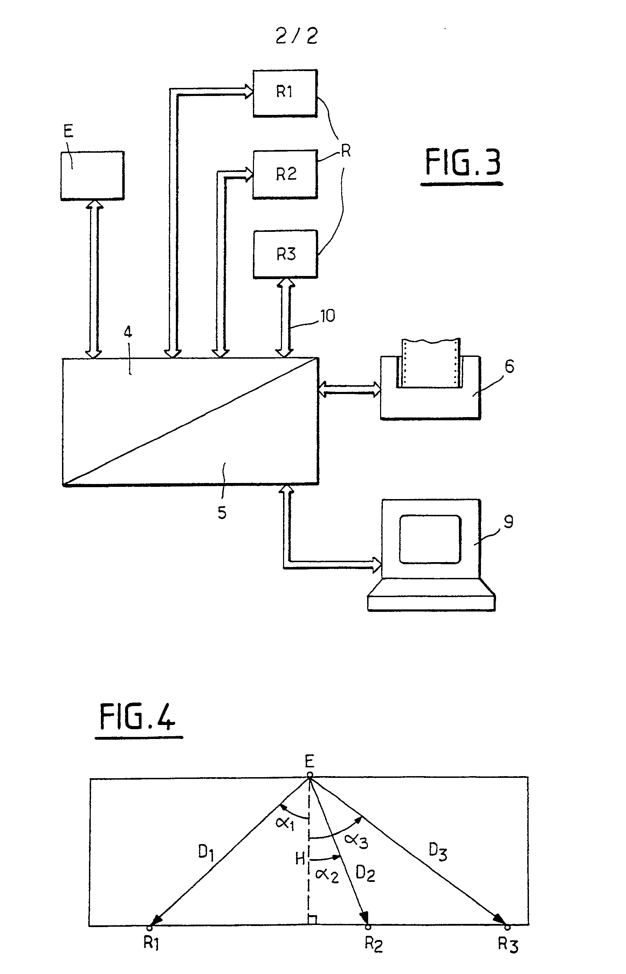

Pour compléter cette description, la figure 3 est un schéma de principe qui montre les différentes liaisons entre les composants du système selon l'invention.

Les transducteurs émetteurs et récepteurs E et R sont reliés à un ensemble électronique 4, connu en soi, qui permet l'émission des trains d'ondes ultrasoniques et mesure le temps de transit de ceux-ci entre deux transducteurs.

L'ensemble de mesure selon l'invention comporte également un calculateur 5 relié à l'ensemble électronique 4 et à un ou plusieurs dispositifs d'entrée et/ou de sortie de résultats tels qu'un dispositif d'impression 6 et/ou un ensemble 9 moniteur et clavier.

De façon avantageuse mais non limitative, une armoire 8 réunit les différents composants qui viennent d'être décrits, à savoir: l'ensemble électronique 4, le calculateur 5 et le dispositif d'impression 6.

Le traitement des temps de propagation des ondes ultrasonores dans le béton a lieu de préférence selon une méthode, dite des antennes, qui consiste à émettre des impulsions d'ondes ultrasonores dans le béton à partir d'un émetteur fixe E et à analyser les ondes longitudinales reçues par plusieurs récepteurs, trois par exemple R1, R2, R3 situés dans un même plan horizontal et alignés (cf. figure 4).

Il est mesuré séquentiellement le temps de parcours des ondes longitudinales arrivant sur chaque récepteur R1 à R3. Ce temps est caractéristique de la compacité du béton et donc de sa résistance.

Par ailleurs, connaissant la distance D1, D2, D3 entre l'émetteur et chaque récepteur, il est calculé la vitesse $V_i$ du son en appliquant la relation suivante: $V_i = \dfrac{D_i}{T_i}$ (i = 1 à 3 dans le cas de la figure 4).

Dans le cas de béton précontraint, soulignons que les ondes étant longitudinales, c'est-à-dire perpendiculaires aux torons de précontrainte, ceux-ci ne perturberont pas les mesures.

Le procédé selon la présente invention est basé sur la méthode précitée et il consiste plus précisément à:
– placer en des endroits prédéterminés d'un moule 1 des transducteurs, grâce à des dispositifs de fixation 30. Un émetteur E étant placé en face d'au moins un récepteur R. Si plusieurs récepteurs sont utilisés, ils seront de préférence alignés:
– faire émettre par l'émetteur E des impulsions d'ondes longitudinales dans une zone incluant tous les récepteurs, cette zone étant aussi vaste que possible afin de souder une large partie de l'élément à mesurer,
– mesurer successivement sur chaque récepteur le temps de parcours d'un train d'onde stabilisé,
– convertir lesdites valeurs de temps selon la méthode dite des antennes, afin d'obtenir des vitesses de propagation des ondes longitudinales,
– comparer chaque valeur de vitesse à un seuil,
– éliminer la ou les vitesses supérieures à un certain seuil et, s'il reste plusieurs valeurs de vitesse, en faire la moyenne arithmétique, sinon conserver l'unique valeur de vitesse tolérable,
– comparer ladite moyenne ou ladite valeur à une valeur prédéfinie qui, grâce à une certaine corrélation, permet de déterminer une caractéristique physique du béton telle que sa compacité,
– arrêter le cycle de mesure et éventuellement le cycle d'étuvage du béton lorsque la valeur de vitesse prédéfinie correspond à une compacité souhaitée du béton, sinon recommencer le procédé depuis l'étape d'émission des trains d'ondes, à des intervalles de temps prédéfinis.

Il existe plusieurs éléments paramétrables dans le procédé selon l'invention, parmi lesquels la position des transducteurs, le temps de stabilisation des trains d'ondes sur chaque récepteur, les différents seuils: vitesse éliminatoire, vitesse d'arrêt du cycle de mesure, mais aussi temps entre deux cycles de mesure...

En outre, l'exemple d'application qui vient d'être décrit permet d'avoir des valeurs de compacité du béton en continu, pendant un cycle d'étuvage, de mieux analyser les durées d'étuvage, et donc d'optimiser les cycles d'étuvage. Rappelons que l'étuvage représente un gain de coût de production très important, car il permet d'atteindre très rapidement un durcissement du béton élevé.

Il résulte de ce qui précède que le procédé et le dispositif selon la présente invention sont très adaptatifs et permettent d'envisager d'autres évolutions et perfectionnements sans sortir du cadre de l'invention.

## Revendications

1. Dispositif de contrôle non destructif pour des éléments en béton, destiné notamment à déterminer une caractéristique mécanique liée à la vitesse de propagation d'ondes ultrasonores se propageant dans le béton, comprenant un moule (1) destiné à contenir le béton, caractérisé en ce qu'il comprend en outre:
   – des transducteurs à ultrasons (E, R) fixés sur le moule, un transducteur-émetteur (E) étant placé sur une paroi du moule faisant face à une autre paroi supportant plusieurs transducteurs-récepteurs (R1, R2, R3);
   – un dispositif de fixation (30) associé à chaque transducteur (E, R) afin de le maintenir contre une des parois du moule (1);
   – un ensemble électronique (4) relié à chacun des transducteurs (2, 3) et destiné à déterminer le temps de propagation des ondes se propageant dans le béton entre le transducteur-émetteur (E) et les transducteurs-récepteurs (R);

– un calculateur programmable (5) relié en entrée à l'ensemble électronique (4) et en sortie à un dispositif de visualisation (8, 9), destiné à traiter lesdits temps de propagation des ondes afin de fournir en continu des données relatives à l'évolution des caractéristiques de l'élément en béton, dont notamment sa résistance mécanique.

2. Ensemble selon la revendication 1, caractérisé en ce que plusieurs transducteurs-récepteurs (R) sont fixés en des endroits prédéterminés sur une première paroi du moule et en ce qu'un seul émetteur (E) est prévu sur la paroi faisant face à ladite première paroi.

3. Ensemble selon la revendication 2, caractérisé en ce que l'ensemble électronique (4) mesure et enregistre successivement les temps stabilisés de parcours des trains d'ondes entre l'émetteur et chacun des récepteurs.

4. Ensemble selon l'une quelconque des revendications précédentes, caractérisé en ce que ledit calculateur programmable (5) détermine la vitesse de propagation de l'onde entre l'émetteur et chacun des récepteurs, élimine les valeurs de vitesse n'appartenant pas à une plage prédéfinie, calcule la vitesse moyenne des vitesses non éliminées, à des intervalles de temps prédéterminés et ce jusqu'à l'obtention d'une valeur de vitesse moyenne prédéfinie.

5. Ensemble selon la revendication 4, caractérisé en ce que les transducteurs sont fixés sur le moule de façon que leur surface "active" soit située dans le plan de la surface interne du moule.

6. Ensemble selon la revendication 5 caractérisé en ce que lesdits transducteurs sont fixés sur le moule grâce à des dispositifs de fixation (30) constitués chacun:

– d'un corps tubulaire (33) entourant et positionnant chacun desdits transducteurs (E, R),
– d'une étoupe (31) coaxiale au corps, entourant en partie le transducteur et axialement en appui sur ledit corps,
– d'un presse-étoupe (32) tubulaire coaxial au corps interposé entre ladite étoupe (31) et un bouchon de serrage (34) vissé sur ledit corps.

7. Ensemble selon l'une quelconque des revendications précédentes caractérisé en ce qu'un film silicone est fixé sur chaque transducteur (E, R) pour être utilisé comme couplant.

8. Procédé de contrôle non destructif de produits en béton utilisant un dispositif de contrôle non destructif pour des éléments en béton, destiné à déterminer une caractéristique mécanique liée à la vitesse de propagation d'ondes ultrasonores se propageant dans le béton, caractérisé en ce qu'il comporte les étapes suivantes:

a) - placer en des endroits prédéterminés sur un moule (1) destiné à recevoir le béton plusieurs transducteurs à ultrasons, l'un étant émetteur (E) et les autres étant récepteurs (R);
b) - faire émettre par ledit transducteur-émetteur (E) des trains d'ondes successifs;
c) - mesurer successivement les temps de propagation stabilisés entre l'émetteur et chaque récepteur;
d) - convertir les temps en vitesse;
e) - traiter lesdites valeurs de vitesse;
f) - établir une corrélation entre les valeurs de vitesse et certaines caractéristiques physiques du béton; et
- répéter les étapes b) à f) jusqu'à obtention d'une caractéristique physique prédéterminée du béton.

9. Procédé selon la revendication 8, caractérisé en ce que le traitement des valeurs de vitesse consiste à:

– comparer chaque vitesse de propagation à une valeur de seuil;
– éliminer la ou les vitesses supérieures à un certain seuil, et
– s'il reste plusieurs valeurs de vitesse, en faire la moyenne arithmétique, sinon conserver ladite valeur; et
– comparer ladite moyenne ou ladite valeur à une valeur prédéfinie.

EP 0 448 896 A1

FIG.1

4

6

8

1

E

R1

R2

R3

10

FIG.2

34

30

1

35

36

32

37

31

E,R

10

33

7

## FIG.3

## FIG.4

Office européen
des brevets

# RAPPORT DE RECHERCHE EUROPEENNE

Numero de la demande

EP 90 40 3706

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | GB-A-2 059 588 (D.L. SUTTON) * Page de garde * --- | 1 | G 01 N 29/18 |
| A | N.T.I.S., 8 février 1983, PAT-APPL-6,400,571; "Composite fiber/matrix stability measurement using ultrasound" * Figure * --- | 1 | |
| A | PATENT ABSTRACTS OF JAPAN, vol. 9, no. 141 (P-364)[1864], 15 juin 1985; & JP-A-60 20 147 (ASAKA EMU AARA K.K.) 01-02-1985 * Figures * --- | 1 | |
| A | EP-A-0 199 375 (G.C. McKINNON) * Résumé * --- | 1 | |
| A | FR-A-2 467 400 (ELF AQUITAINE) * Revendication 1 * ----- | 1 | |

DOMAINES TECHNIQUES
RECHERCHES (Int. Cl.5)

G 01 N

Le présent rapport a été établi pour toutes les revendications

| Lieu de la recherche | Date d'achèvement de la recherche | Examinateur |
|---|---|---|
| LA HAYE | 21-06-1991 | DUCHATELLIER M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

                                                
& : membre de la même famille, document correspondant

EPO FORM 1503 03.82 (P0402)